Europäisches Patentamt

European Patent Office

Office européen des brevets

(19)

(11) Numéro de publication: **0 099 872**
**A2**

(12) **DEMANDE DE BREVET EUROPEEN**

(21) Numéro de dépôt: **83870072.2**

(22) Date de dépôt: **15.07.83**

(51) Int. Cl.³: **A 61 N 1/16**

(30) Priorité: **19.07.82 BE 208622**

(43) Date de publication de la demande:
**01.02.84 Bulletin 84/5**

(84) Etats contractants désignés:
**AT CH DE FR GB IT LI LU NL SE**

(71) Demandeur: **Kunnen, Walter Antoon**
**323 Prins Boudewijnlaan**
**B-2610 Wilrijk(BE)**

(71) Demandeur: **ARCHIBO-BIOLOGICA, Naamloze**
**Vennootschap**
**Kasteelstraat 15**
**B-3671 Maaseik(BE)**

(72) Inventeur: **Kunnen, Walter Antoon**
**Prins Boudewijnlaan, 323**
**B-2610 Wilrijk(BE)**

(74) Mandataire: **Pieraerts, Jacques et al,**
**Bureau Gevers S.A. rue de Livourne 7, Bte. 1**
**B-1050 Bruxelles(BE)**

(54) Procédé de protection des êtres vivants contre des rayonne-menus ou champs électromagnétiques nuisibles et élément utilisé à cet effet.

(57) Procédé en vue de protéger des êtres vivants contre des rayonnements et des champs électromagnétiques perturbés, en particulier en vue de protéger des êtres humains dans un espace d'habitation, caractérisé en ce qu'on détermine d'abord la longueur d'onde et la direction du champ magnétique perturbé, considéré comme nuisible, et en ce qu'on dispose perpendiculairement'au vecteur rectiligne de ce dernier au moins une petite "brique" possédant des "propriétés d'antenne", destinée à diriger les lignes de force de ce champ magnétique perturbé en dehors de l'espace habité précité, ou, à tout le moins, hors d'une zone déterminée de celui-ci.

EP 0 099 872 A2

"Procédé de protection des êtres vivants contre des rayonnements ou champs électromagnétiques nuisibles et élément utilisé à cet effet".

L'invention se rapporte à un procédé en vue de protéger les êtres vivants contre des rayonnements et des champs électromagnétiques perturbés, en particulier en vue de protéger des êtres humains dans un espace habité.

Quoique la conviction se renforce qu'une série de phénomènes pathologiques, dont certains sont à la base de dérangements sérieux, trouvent leur origine dans le terrain ou le sol sur lequel nous séjournons une partie importante de notre temps, aucun moyen efficace permettant de remédier à ces phénomènes liés au sol n'a été proposé.

Par l'expression " séjourner une partie importante de son temps" il faut entendre que des êtres vivants, essentiellement des humains, occupent une partie déterminée d'un espace d'habitation, un bureau, un atelier, chambre à coucher ou pièce de séjour.

Le demandeur a pu constater à diverses reprises que certains endroits d'une maison subissent l'influence de modifications du champ électromagnétique, notamment en raison de la présence d'une source d'eau ou de câbles de haute tension.

Les modifications et les déviations du champ magnétique dans lequel nous vivons sont à la base des perturbations pathologiques citées plus haut.

D'autres causes de dérangement sont, par exemple, les réfrigérateurs, les appareils de télévision, les boilers, etc., qui perturbent les fonctions humaines bioélectriques de faible tension.

Ces phénomènes, comme bien d'autres dont la nature est mieux connue et mieux identifiée à l'heure actuelle, expliquent les causes de maladies attribuées depuis longtemps à certaines maisons ou à certains endroits.

L'invention a pour but de procurer un procédé permettant de remédier aux influences nuisibles des phénomènes précités sur les êtres humains.

A cet effet, on détermine d'abord la longueur d'onde et la direction du champ magnétique perturbé, considéré comme nuisible , et on dispose perpendiculairement au vecteur rectiligne de ce dernier, une petite "brique" possédant des "propriétés d'antenne",afin de diriger les lignes de force dudit champ magnétique perturbé en dehors de l'espace d'habitation précité ou, à tout le moins, hors d'une zone déterminée de celui-ci.

L'invention a également trait aux éléments destinés à appliquer le procédé selon l'invention.

Un tel élément est avantageusement constitué par deux tiges métalliques possédant de bonnes "propriétés d'antenne". De préférence ces tiges sont constituées d'aluminium ou d'un alliage aluminium-cuivre. Conformément à l'invention ces tiges sont enrobées d'un moyen de protection contre l'oxydation.

Conformément à l'invention, les tiges sont noyées dans un milieu à base de ciment et présentent des dimensions d'environ 27,50 cm sur 4,5 cm, le choix de la troisième dimension demeurant arbitraire.

D'autres détails et avantages de l'invention ressortiront de la description qui sera donnée ci-après d'un procédé en vue de protéger des êtres vivants contre des rayonnements électromagnétiques nuisibles, en particulier en vue de protéger des êtres humains, et des éléments utilisés à cet effet.

Cette description n'est donnée qu'à titre d'exemple et ne limite pas l'invention.

Le procédé selon l'invention, a pour base une série de phénomènes qui peuvent être observés chez des êtres vivants, tels que la fatigue, l'insomnie, la perte d'appétit, l'apparition de phénomènes dégénératifs plus ou moins généralisés, et qui trouvent leur cause dans la présence, dans un espace d'habitation, par exemple, d'un champ électromagnétique modifié de telle sorte que des êtres vivants séjournant en des endroits déterminés de cet espace subissent l'influence défavorable et renforcée de ces champs électromagnétiques perturbés.

Il n'est donc pas étonnant que lorsque que l'on constate la présence d'un tel champ électromagnétique perturbé dans une chambre à coucher, a fortiori à travers le lit, des effets nuisibles observés dans une telle situation peuvent agir plusieurs heures par jour sur une même personne.

En dehors des êtres vivants, il a également été constaté que certains matériaux de construction, et les bâtiments eux-mêmes subissent l'influence des champs magnétiques perturbés.

L'invention, qui a essentiellement pour but de protéger des êtres vivants contre les influences précitées prévoit le détournement des champs électromagnétiques perturbés.

A cet effet, sont proposés un procédé et un élément utilisés dans le cadre de celui-ci, qui atteignent parfaitement le but visé.

Conformément à l'invention, on détermine d'abord la longueur d'onde et la direction du champ électromagnétique perturbé considéré comme nuisible.

On fait usage, de préférence, de deux tiges métalliques qui sont noyées ou non dans un élément réalisé à base de ciment.

Les tiges métalliques consistent en aluminium ou en un alliage aluminium-cuivre et provoquent la déviation, grâce à leur "propriétés d'antenne", des rayonnements nuisibles.

L'élément dans lequel sont noyées les tiges métalliques a essentiellement pour but de protéger ces tiges sensibles aux lignes de force contre l'oxydation.

Il est également évident que, dans certains cas, d'autres dimensions peuvent également être prescrites utilement.

Il est enfin évident que l'invention n'est pas limitée à la forme d'exécution décrite ci-dessus et que bien des modifications pourraient y être apportées en ce qui concerne la forme, la disposition, la situation et le nombre des éléments qui entrent en ligne de compte pour la bonne réalisation de l'invention.

REVENDICATIONS.

1. Procédé en vue de protéger des êtres vivants contre des rayonnements et des champs électromagnétiques perturbés, en particulier en vue de protéger des êtres humains dans un espace d'habitation, caractérisé en ce qu'on détermine d'abord la longueur d'onde et la direction du champ magnétique perturbé, considéré comme nuisible, et en ce qu'on dispose perpendiculairement au vecteur rectiligne de ce dernier au moins une petite "brique" possédant des "propriétés d'antenne", destinée à diriger les lignes de force de ce champ magnétique perturbé en dehors de l'espace habité précité, ou, à tout le moins, hors d'une zone déterminée de celui-ci.

2. Elément pour l'application du procédé selon la revendication 1, caractérisé en ce qu'il est constitué par deux tiges métalliques possédant de bonnes "propriétés d'antenne".

3. Elément selon la revendication 2, caractérisé en ce que les tiges précitées sont constituées d'aluminium ou d'un alliage offrant des propriétés analogues.

4. Elément selon l'une quelconque des revendications 2 ou 3, caractérisé en ce que les tiges précitées sont noyées dans un milieu à base de ciment, par exemple, protégeant contre l'oxydation.

5. Elément selon l'une quelconque des revendications 2 à 4, caractérisé en ce que des dimensions avantageuses pour la petite "brique" précitée sont d'environ 27,50 cm sur environ 4,5 cm, le choix de la troisième dimension demeurant arbitraire.